# FASCICULE DE BREVET EUROPEEN

(11) **EP 4 176 255 B1**
(45) Date de publication et mention de la délivrance du brevet: **10.07.2024**
(21) Numéro de dépôt: 21737453.7
(22) Date de dépôt: 29.06.2021
(51) Int. Cl.: G01N 33/00

(54) **SYSTÈME DE CAPTATION CHIMIQUE À ÉTALONNAGE INTÉGRÉ ET PROCÉDÉ D'UTILISATION D'UN TEL SYSTÈME**
CHEMISCHES ERFASSUNGSSYSTEM MIT INTEGRIERTER KALIBRIERUNG UND VERFAHREN ZUR VERWENDUNG SOLCH EINES SYSTEMS
CHEMICAL CAPTURE SYSTEM WITH INTEGRATED CALIBRATION AND METHOD FOR USING SUCH A SYSTEM

(30) Priorité: 01.07.2020 FR 2006965
(43) Date de publication de la demande: 10.05.2023
(73) Titulaire: ELLONA, 31400 Toulouse (FR)
(72) Inventeur: ROMANYTSIA, Ivan, 31300 TOULOUSE (FR)
(74) Mandataire: Argyma
(86) Numéro de dépôt international: PCT/EP2021/067920
(87) Numéro de publication internationale: WO 2022/002979

(56) Documents cités:
- DE-A1- 102017 002 764
- US-A1- 2015 369 784
- US-A1- 2019 265 183

## Description

### Domaine technique

La présente invention concerne le domaine des systèmes de captation chimique à étalonnage intégré et l'utilisation de tels systèmes.

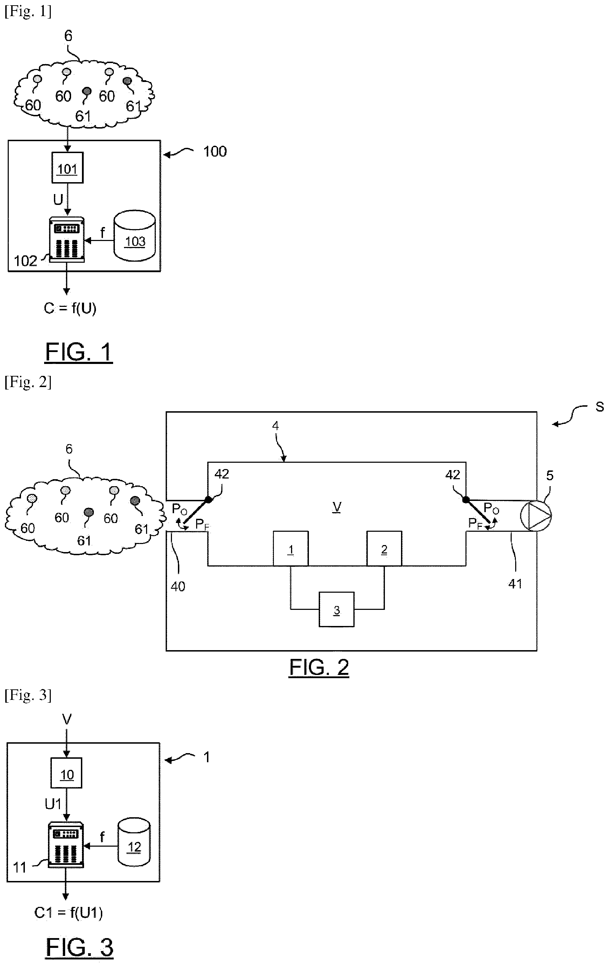

De manière connue, en référence à la [Fig.1], un capteur chimique 100 est configuré pour mesurer un signal physique C, tel que la concentration, d'un ou plusieurs composé(s) chimique(s) 60, 61 d'un mélange gazeux 6, suivant sa sensibilité. Pour cela, un capteur chimique 100 comprend un élément de mesure 101 configuré pour émettre un signal électrique U qui est fonction de la quantité du mélange gazeux 6 et un élément de calcul 102 de la concentration C à partir du signal électrique U émis et d'une fonction de conversion f, propre au capteur chimique 100 et stockée dans une base de données 103, suivant la relation suivante : C=f(U).

De manière connue, un capteur chimique 100 doit être étalonné avant sa première utilisation. Pour cela, le capteur chimique 100 est placé dans plusieurs milieux étalons correspondant à des mélanges gazeux connus dont les concentrations sont connues. L'écart entre la concentration C mesurée par le capteur chimique 100 et la concentration en composés chimiques réelle de chaque milieu étalon est corrigé en modifiant la fonction de conversion f du capteur chimique 100 afin de réduire cet écart.

Dans les faits, la réponse d'un capteur chimique 100 dérive au cours de son utilisation, notamment lorsque le capteur chimique 100 se présente sous la forme d'un capteur semi-conducteur ou d'un capteur électrochimique. Plus précisément, pour un mélange gazeux 6 identique, l'élément de mesure 101 du capteur chimique 100 émet un signal électrique U différent au fur et à mesure de son utilisation. En effet, dans le cas d'un capteur semi-conducteur, également nommé « capteur MOX » ou « capteur MOS », l'élément de mesure 101 comprend une couche d'oxyde métallique chauffée par un élément chauffant, sur laquelle viennent se fixer les composés chimiques 60, 61 du mélange gazeux 6, ce phénomène étant connu sous le terme « d'adsorption ». L'élément de mesure 101 comprend en outre des électrodes de mesure configurées pour émettre un signal électrique U correspondant à une conductivité électrique de la couche d'oxyde métallique. Or, au fur et à mesure de l'utilisation du capteur semi-conducteur, l'adsorption des composés chimiques 60, 61 obstrue les pores de la couche d'oxyde métallique, ce qui modifie le signal électrique U émis.

Un tel capteur 100 doit ainsi être régulièrement étalonné pour que la concentration C mesurée reste fiable et précise. En pratique, l'étalonnage est réalisé en ajustant la fonction de conversion f pour qu'elle prenne en compte la dérive du signal électrique U dans le calcul de la concentration C. Ceci nécessite de quantifier la dérive du signal électrique U, ce qui est réalisé en plaçant le capteur 100 dans plusieurs milieux étalons et en mesurant l'écart entre la concentration C mesurée par le capteur chimique 100 et la concentration en composés chimiques réelle de chaque milieu étalon. Un tel étalonnage au cours de la vie du capteur 100 nécessite de désinstaller puis de réinstaller le capteur 100 au contact du mélange gazeux 6, ce qui est coûteux en temps et en ressources. En outre, en cas d'oubli ou de retard pour réaliser l'étalonnage ou encore de dérèglement imprévu du capteur 100, une concentration C faussée peut être mesurée sans le savoir.

De manière incidente, il est connu un système de captation d'ozone à étalonnage intégré comprenant une chambre ainsi qu'un capteur électrochimique d'ozone et un générateur d'ozone placés dans la chambre. Lors d'une phase de mesure, la chambre est ouverte de manière à guider le mélange gazeux dont on souhaite mesurer la concentration en ozone vers le capteur. Lors d'une phase d'étalonnage, la chambre est fermée et l'ozone restant dans la chambre est décomposé par contact avec les parois de la chambre. Le générateur d'ozone est alors configuré pour émettre une concentration connue d'ozone dans la chambre fermée qui est mesurée par le capteur électrochimique. L'écart entre la concentration d'ozone mesurée par le capteur électrochimique et émise par le générateur d'ozone permet d'étalonner le capteur. Un tel système permet avantageusement de réaliser l'étalonnage sans déplacer le capteur. Toutefois, il présente l'inconvénient de n'être opérable que pour le cas particulier d'un capteur sensible uniquement à l'ozone. En effet, pour un capteur sensible également aux composés organiques volatils, d'abréviation « COV », des COV peuvent rester piégés dans la chambre fermée lors de l'étalonnage. De tels COV faussent l'étalonnage car ils ne se décomposent pas comme l'ozone, sont présents en concentration inconnue et le capteur les mesure au même titre que l'ozone sans distinction.

Il est également connu par la demande de brevet US2019265183A1 un système de captation de gaz à étalonnage intégré comportant une enceinte dans laquelle sont montés un capteur, une plaque chauffante pour le réinitialiser, une source lumineuse et un élément chauffant. L'élément chauffant permet de décomposer l'ozone dans l'enceinte pour calibrer le capteur en ligne de base et la source lumineuse permet de générer de l'ozone en concentration connue pour finir la calibration. Les éléments dédiés à l'étalonnage présentent l'inconvénient d'augmenter l'encombrement du système, particulièrement en période de mesure où ils sont inactifs, et d'augmenter le risque de maintenance. De plus, en cas de dérèglement imprévu du capteur, une concentration faussée peut être mesurée sans le savoir.

Par ailleurs, il est connu par la demande de brevet US2015369784A1 d'étalonner un capteur MOX en comparant sa mesure à celle d'un capteur à photoionisation, monté sur un circuit différent et allumé uniquement en période d'étalonnage. Une telle solution présente les mêmes inconvénients que précédemment.

L'invention vise ainsi à éliminer au moins certains des inconvénients cités liés à l'étalonnage d'un capteur chimique.

### PRESENTATION DE L'INVENTION

L'invention concerne un procédé d'utilisation d'un système de captation chimique à étalonnage intégré pour la mesure d'un mélange gazeux, ledit système comprenant :
- une chambre délimitant un volume intérieur et comprenant au moins une ouverture de mise en communication fluidique du mélange gazeux et du volume intérieur et au moins un organe de fermeture de ladite ouverture monté mobile entre une position fermée délimitant un volume intérieur fermé et une position ouverte délimitant un volume intérieur ouvert,
- au moins un capteur chimique, dit capteur à étalonner, positionné dans le volume intérieur de la chambre et comprenant un élément de mesure configuré pour émettre un signal électrique qui est fonction de la quantité d'au moins un composé chimique prédéterminé dans le volume intérieur, ledit capteur à étalonner comprenant un élément de calcul d'un premier signal physique (C1) dudit composé chimique à partir dudit signal électrique (U1) et d'une fonction de conversion (f) propre audit capteur à étalonner suivant la relation suivante : C1 = f(U1),
- au moins un capteur à photoionisation positionné dans le volume intérieur, réputé non sujet à la dérive et configuré, d'une part, pour émettre des rayons ultraviolets de manière à photoioniser au moins un composé chimique prédéterminé dans le volume intérieur, et d'autre part, pour déterminer un deuxième signal physique qui est fonction de la quantité dudit composé chimique photoionisé, et
- au moins un dispositif de pilotage,
- procédé dans lequel, lors d'au moins une étape de mesure :
   - le dispositif de pilotage commande le déplacement de l'organe de fermeture en position ouverte de manière à ce que la chambre délimite un volume intérieur ouvert dans lequel pénètre le mélange gazeux,
   - le capteur à étalonner et le capteur à phototoionisation mesurent respectivement au moins un premier signal physique de mesure et au moins un deuxième signal physique de mesure du mélange gazeux, et
   - le dispositif de pilotage identifie le mélange gazeux à partir des signaux physiques de mesure.

L'invention est remarquable en ce que, lors d'au moins une étape d'étalonnage :
- le dispositif de pilotage commande le déplacement de l'organe de fermeture en position fermée de manière à ce que la chambre délimite un volume intérieur fermé,
- lorsque le volume intérieur fermé est exempt de mélange gazeux et d'ozone, le capteur à photoionisation génère de l'ozone dans le volume intérieur fermé en émettant suivant une puissance donnée des rayons ultraviolets configurés pour photoioniser le dioxygène présent,
- le capteur à étalonner mesure un signal physique d'étalonnage de l'ozone généré,
- le dispositif de pilotage calcule l'écart entre le signal physique d'étalonnage et un signal physique de référence et
- si l'écart est supérieur à un écart de référence, le dispositif de pilotage détermine une fonction de conversion optimisée à partir dudit écart pour le capteur à étalonner, de manière à l'étalonner.

Grâce à l'invention, il est possible d'utiliser un système de captation chimique pour remplir deux fonctions, à savoir mesurer un mélange gazeux et étalonner un capteur chimique. Un tel étalonnage interne du capteur au sein du système de captation chimique évite une manipulation humaine pour déplacer le capteur dans des milieux étalons connus pour réaliser l'étalonnage, ce qui est plus pratique et plus rapide, une telle manipulation devant de surcroît être répétée régulièrement. L'étalonnage est de plus réalisé en utilisant de manière innovante les moyens de mesure, et notamment le capteur à photoionisation, ce qui limite l'encombrement et le coût du système de captation chimique. Plus précisément, le capteur à photoionisation permettant de compléter la mesure du capteur à étalonner lors d'une étape de mesure, est judicieusement utilisé suivant une deuxième fonction comme un générateur d'ozone lors d'une étape d'étalonnage, en photoionisant le dioxygène naturellement présent dans l'air. Autrement dit, le capteur à photoionisation permet, outre sa fonction de capteur, de créer dans le volume intérieur fermé un milieu d'étalonnage connu avec une certaine quantité d'ozone pour étalonner le capteur à étalonner, c'est-à-dire une concentration d'étalonnage prédéterminée. Un tel capteur à photoionisation est fiable car non sujet à la dérive. La mise en oeuvre de l'étape d'étalonnage à la suite d'une étape de mesure, et inversement, est en outre simple et rapide, régie par le dispositif de pilotage qui déplace l'organe de fermeture de la position ouverte à la position fermée, et inversement.

Selon un aspect, le système comprenant au moins un dispositif d'élimination du mélange gazeux dans le volume intérieur, lors de l'étape d'étalonnage, le dispositif d'élimination élimine le mélange gazeux éventuellement rémanent dans le volume intérieur avant la génération d'ozone. Le dispositif d'élimination du mélange gazeux permet avantageusement de mettre en oeuvre l'étape d'étalonnage à un instant quelconque, en présence ou non de mélange gazeux, en l'éliminant de manière préliminaire.

Selon un premier aspect de l'invention, la chambre comprenant au moins une ouverture d'entrée et au moins une ouverture de sortie, le système de captation comprenant au moins un organe de ventilation monté dans la chambre, de préférence au niveau de l'ouverture de sortie, et au moins un organe de filtrage du mélange gazeux monté mobile au niveau de l'ouverture d'entrée entre une position déployée et une position escamotée et formant avec l'organe de ventilation le dispositif d'élimination du mélange gazeux, l'élimination du mélange gazeux lors de l'étape d'étalonnage est mise en oeuvre dans le volume intérieur ouvert par le dispositif de pilotage en commandant conjointement le déplacement de l'organe de filtrage en position déployée et l'activation de l'organe de ventilation, de manière à éviter l'admission de mélange gazeux dans le volume intérieur ouvert et à évacuer le mélange gazeux éventuellement rémanent hors du volume intérieur ouvert.

Une telle élimination du mélange gazeux dans le volume intérieur ouvert de la chambre est simple et pratique à mettre en oeuvre, utilisant des moyens réduits à faible coût, à savoir l'organe de filtrage, conjointement aux moyens de circulation et de renouvellement du mélange gazeux, à savoir l'organe de ventilation. Une telle élimination est en outre efficace et rapide, en particulier pour une chambre de taille réduite, le mélange gazeux étant bloqué au niveau de l'ouverture d'entrée, et évacué au niveau de l'ouverture de sortie.

Selon un deuxième aspect de l'invention, le capteur à photoionisation formant le dispositif d'élimination du mélange gazeux, l'élimination du mélange gazeux lors de l'étape d'étalonnage est mise en oeuvre dans le volume intérieur fermé par le capteur à photoionisation en émettant des rayons ultraviolets configurés, d'une part, pour générer de l'ozone par photoionisation du dioxygène présent afin de réagir par ozonolyse avec le mélange gazeux éventuellement rémanent dans le volume intérieur fermé, et d'autre part, pour photoioniser ledit mélange gazeux.

Une telle élimination du mélange gazeux dans le volume intérieur fermé de la chambre utilise avantageusement le capteur à photoionisation suivant une troisième fonction de dispositif d'élimination du mélange gazeux, outre sa première fonction de capteur de mesure et sa deuxième fonction de générateur d'ozone. Une telle élimination du mélange gazeux est également efficace et rapide, par l'action combinée de l'ozonolyse et de la photoionisation. Autrement dit, le capteur à photoionisation permet d'éliminer le mélange gazeux de deux façons via les rayons ultraviolets qu'il émet : majoritairement d'une façon indirecte en générant de l'ozone qui réagit avec le mélange gazeux par ozonolyse, et minoritairement d'une façon directe par photoionisation du mélange gazeux. Le mélange gazeux ainsi éliminé par photoionisation et ozonolyse n'est avantageusement pas détecté par le capteur à étalonner.

Selon un aspect de l'invention, lors de l'étape de mesure, le premier signal physique de mesure et le deuxième signal physique de mesure sont mesurés simultanément. La concentration du mélange gazeux pouvant évoluer avec le temps, ceci garantit que le mélange gazeux mesuré par le capteur à étalonner et le capteur à photoionisation est identique, et donc que leurs mesures peuvent être croisées pour identifier de manière précise et fiable le mélange gazeux à un (ou plusieurs) instant(s) données).

Selon un autre aspect de l'invention, lors d'au moins une étape d'initialisation :
- le dispositif de pilotage commande le déplacement de l'organe de fermeture en position fermée de manière à ce que la chambre délimite un volume intérieur fermé,
- lorsque le volume intérieur fermé est exempt de mélange gazeux et d'ozone, le capteur à photoionisation génère de l'ozone dans le volume intérieur fermé en émettant suivant ladite puissance donnée des rayons ultraviolets configurés pour photoioniser le dioxygène présent et
- le capteur à étalonner mesure ledit signal physique de référence de l'ozone généré.

De manière avantageuse, une telle période d'initialisation permet de déterminer un signal physique de référence formant une source de comparaison fiable et précise pour le signal physique d'étalonnage. En effet, le signal physique de référence est mesuré lorsque le capteur à étalonner est réputé valide. De plus le signal physique de référence est de même nature que le signal physique d'étalonnage, notamment mesuré dans le même milieu - le volume intérieur fermé de la chambre étant exempt du mélange gazeux à mesurer et comprenant de l'ozone en concentration connue et identique, et par le même appareil de mesure - le capteur à étalonner.

De préférence, lors de l'étape d'élimination, le dispositif d'élimination élimine le mélange gazeux éventuellement rémanent dans le volume intérieur, de manière analogue à l'étape d'étalonnage. Ceci permet de mettre l'étape d'initialisation à un instant quelconque, en présence ou non du mélange gazeux.

De préférence, le procédé d'utilisation comprend une unique étape d'initialisation, préliminaire à toute étape de mesure et d'étalonnage. Le signal physique d'étalonnage obtenu sert ainsi de base de comparaison pour toutes les étapes d'étalonnage.

De préférence, le procédé d'utilisation comprend une alternance d'étapes de mesure et d'étapes d'étalonnage. L'intérêt d'un tel système de captation chimique à étalonnage intégré est de mesurer le mélange gazeux, et lorsque nécessaire, d'interrompre la mesure pour réaliser un étalonnage. De préférence, les étapes d'étalonnage sont effectuées régulièrement pour que la dérive du capteur à étalonner reste faible.

L'invention concerne également un système de captation chimique à étalonnage intégré pour la mesure d'un mélange gazeux, ledit système comprenant :
- une chambre délimitant un volume intérieur et comprenant au moins une ouverture de mise en communication fluidique du mélange gazeux et du volume intérieur et au moins un organe de fermeture de ladite ouverture monté mobile entre une position fermée délimitant un volume intérieur fermé et une position ouverte délimitant un volume intérieur ouvert,
- au moins un capteur chimique, dit capteur à étalonner, positionné dans le volume intérieur de la chambre et comprenant un élément de mesure configuré pour émettre un signal électrique qui est fonction de la quantité d'au moins un composé chimique prédéterminé dans le volume intérieur, ledit capteur à étalonner comprenant un élément de calcul d'un premier signal physique (C1) dudit composé chimique à partir dudit signal électrique (U1) et d'une fonction de conversion (f) propre audit capteur à étalonner suivant la relation suivante : C1 = f(U1), ledit capteur à étalonner étant en particulier configuré pour déterminer, respectivement lors d'une étape de mesure et lors d'une étape d'étalonnage du procédé d'utilisation tel que décrit précédemment, un premier signal physique de mesure du mélange gazeux dans le volume intérieur ouvert et un signal physique d'étalonnage de l'ozone dans le volume intérieur fermé,
- au moins un capteur à photoionisation positionné dans le volume intérieur et configuré, d'une part, pour émettre des rayons ultraviolets de manière à photoioniser au moins un composé chimique prédéterminé dans le volume intérieur, et d'autre part, pour déterminer un deuxième signal physique qui est fonction de la quantité dudit composé chimique photoionisé, ledit capteur à photoionisation étant en particulier configuré, lors de l'étape de mesure, pour déterminer un deuxième signal physique de mesure du mélange gazeux dans le volume intérieur ouvert et, lors de l'étape d'étalonnage, pour émettre des rayons ultraviolets suivant une puissance donnée pour photoioniser le dioxygène en ozone dans le volume intérieur fermé, et
- au moins un dispositif de pilotage configuré pour commander le déplacement de l'organe de fermeture en position ouverte lors de l'étape de mesure, de manière à ce que la chambre délimite un volume intérieur ouvert, et en position fermée lors de l'étape d'étalonnage, de manière à ce que la chambre délimite un volume intérieur fermé, ledit dispositif de pilotage étant configuré :
   - lors de l'étape de mesure, pour identifier le mélange gazeux à partir du premier et du deuxième signal physique de mesure et,
   - lors de l'étape d'étalonnage, pour calculer l'écart entre le signal physique d'étalonnage et un signal physique de référence et, si l'écart est supérieur à un écart de référence, déterminer une fonction de conversion optimisée pour le capteur à étalonner, de manière à l'étalonner.

Un tel système de captation chimique à étalonnage intégré forme avantageusement un ensemble unitaire double fonction, à savoir pour la mesure et pour l'étalonnage. Un tel système de captation présente en outre un encombrement et un coût limités, utilisant les mêmes moyens pour la mesure et pour l'étalonnage. En particulier, le capteur à photoionisation est utilisé d'une part comme capteur de mesure et d'autre part comme générateur d'ozone à partir du dioxygène naturellement présent dans l'air.

De préférence, le système comprend en outre au moins un dispositif d'élimination du mélange gazeux dans le volume intérieur de la chambre, afin de pouvoir mettre en oeuvre l'étalonnage à un instant quelconque en présence ou non de mélange gazeux rémanent dans le volume intérieur.

Selon un aspect de l'invention, la chambre comprend au moins une ouverture d'entrée, au moins une ouverture de sortie et au moins un organe de fermeture de l'ouverture d'entrée et de l'ouverture de sortie, afin de faciliter la circulation du mélange gazeux dans le volume intérieur ouvert. Une telle chambre comprenant une ouverture d'entrée et une ouverture de sortie permet une mesure fiable et précise du mélange gazeux, lors de l'étape de mesure du procédé d'utilisation. En effet, l'ouverture d'entrée et l'ouverture de sortie génèrent une circulation du mélange gazeux et évitent donc toute stagnation locale du mélange gazeux. Le capteur à étalonner et le capteur à photoionisation mesurent ainsi une concentration globale et non locale du mélange gazeux dans l'air.

Selon un autre aspect de l'invention, le système de captation chimique comprend au moins un organe de ventilation monté dans la chambre, de préférence au niveau de l'ouverture de sortie, pour favoriser le renouvellement du mélange gazeux dans le volume intérieur ouvert. Un tel organe de ventilation améliore ainsi la fiabilité et la précision de la mesure du capteur à étalonner et du capteur à photoionisation.

Selon un aspect préféré, le système de captation chimique comprend au moins un organe de filtrage du mélange gazeux monté mobile au niveau de l'ouverture d'entrée entre une position déployée et une position escamotée. Un tel organe de filtrage forme avantageusement avec l'organe de ventilation le dispositif d'élimination du mélange gazeux. Un tel dispositif d'élimination est peu complexe, de coût limité et permet d'éliminer le mélange gazeux dans un volume intérieur ouvert.

Selon un autre aspect préféré, le capteur à photoionisation forme le dispositif d'élimination du mélange gazeux. Un tel dispositif d'élimination ne présente pas d'encombrement supplémentaire et permet d'éliminer le mélange gazeux dans un volume intérieur fermé.

De préférence, le capteur à étalonner se présente sous la forme d'un capteur non sélectif, sensible au moins au mélange gazeux à mesurer et à l'ozone. Un tel capteur est sujet à la dérive et doit être étalonné régulièrement, ce que permet le système de captation chimique de manière simple et pratique.

Selon un aspect de l'invention, le capteur à étalonner se présente sous la forme d'un capteur semi-conducteur ou d'un capteur électrochimique. Le système de captation chimique est avantageusement adapté pour un grand nombre de capteurs à étalonner de nature et de sensibilité différentes.

### PRESENTATION DES FIGURES

L'invention sera mieux comprise à la lecture de la description qui va suivre, donnée uniquement à titre d'exemple, et se référant aux dessins annexés donnés à titre d'exemples non limitatifs, dans lesquels des références identiques sont données à des objets semblables et sur lesquels :
[Fig.1] La [Fig.1] est une représentation schématique d'un capteur chimique ;
[Fig.2] La [Fig.2] est une représentation schématique du système de captation chimique à étalonnage intégré selon une forme de réalisation de l'invention ;
[Fig.3] La [Fig.3] est une représentation schématique du capteur à étalonner du système de captation chimique de la [Fig.2] ;
[Fig.4] La [Fig.4] est une représentation schématique du capteur à photoionisation du système de captation chimique de la [Fig.2] ;
[Fig.5] La [Fig.5] est une représentation schématique du procédé d'utilisation du système de captation chimique de la [Fig.2] selon un mode de mise en oeuvre de l'invention ;
[Fig.6A] La [Fig.6A] est une représentation schématique du système de captation de la [Fig.2] lors d'une étape de mesure ;
[Fig.6B] La [Fig.6B] est une représentation schématique du capteur à étalonner de la [Fig.3] lors de l'étape de mesure de la [Fig.6A] ;
[Fig.6C] La [Fig.6C] est une représentation schématique du capteur à photoionisation de la [Fig.4] lors de l'étape de mesure de la [Fig.6A] ;
[Fig.7A] La [Fig.7A] est une représentation schématique du système de captation chimique de la [Fig.2] lors d'une étape d'étalonnage ;
[Fig.7B] La [Fig.7B] est une représentation schématique de l'élimination du mélange gazeux lors de l'étape d'étalonnage de la [Fig.7A] selon un premier mode de mise en oeuvre de l'invention ;
[Fig.7C] La [Fig.7C] est une représentation schématique de l'élimination du mélange gazeux lors de l'étape d'étalonnage de la [Fig.7A] selon un deuxième mode de mise en oeuvre de l'invention ;
[Fig.7D] La [Fig.7D] est une représentation schématique du capteur à étalonner de la [Fig.3] lors de l'étape d'étalonnage de la [Fig.7A] ;
[Fig.7E] La [Fig.7E] est une représentation schématique du capteur à photoionisation de la [Fig.4] lors de l'étape d'étalonnage de la [Fig.7A] ;
[Fig.7F] La [Fig.7F] est une représentation schématique du calcul de l'écart entre la concentration mesurée par le capteur à étalonner lors d'une étape d'initialisation et lors de l'étape d'étalonnage et
[Fig.8] La [Fig.8] est une représentation schématique du système de captation chimique à étalonnage intégré de la [Fig.2] lors de l'étape d'initialisation.

Il faut noter que les figures exposent l'invention de manière détaillée pour mettre en oeuvre l'invention, lesdites figures pouvant bien entendu servir à mieux définir l'invention le cas échéant.

### DESCRIPTION DETAILLEE DE L'INVENTION

En référence à la [Fig.2], l'invention concerne un système de captation chimique à étalonnage intégré S permettant suivant une première utilisation l'identification d'un mélange gazeux 6 via la mesure d'un capteur chimique 1, et suivant une deuxième utilisation l'étalonnage dudit capteur chimique 1. A titre d'exemple, le système S est monté dans un bureau et configuré pour identifier un mélange gazeux 6 se présentant sous la forme de polluants, tels que des composés organiques volatils, émis par l'occupation humaine dans l'air ambiant du bureau.

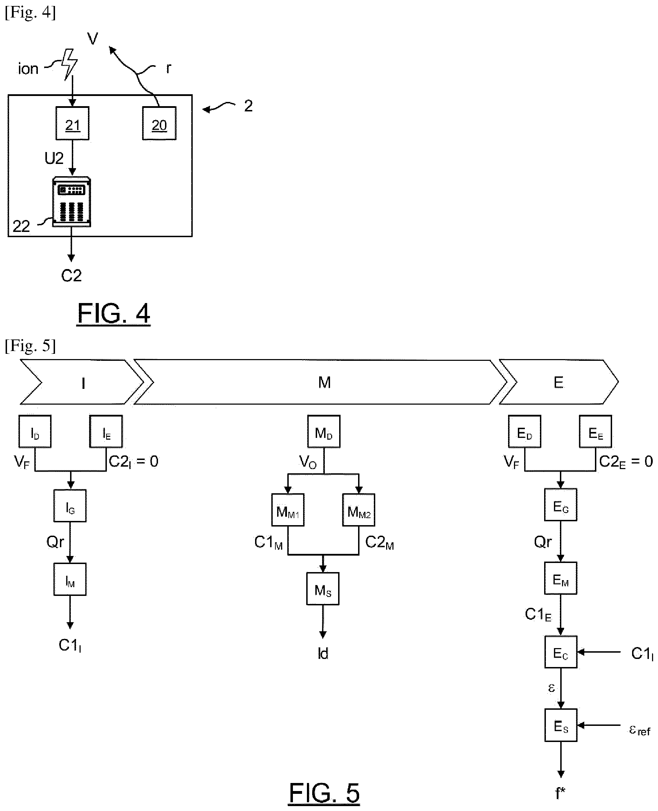

On présente par la suite les caractéristiques structurelles et fonctionnelles du système S puis son procédé d'utilisation, à travers une étape d'initialisation I, une étape de mesure M et une étape d'étalonnage E (voir [Fig.5]).

Selon l'invention et comme illustré sur la [Fig.2], le système de captation chimique S comprend :
- une chambre 4 délimitant un volume intérieur V et comprenant une (ou plusieurs) ouverture(s) 40, 41 et un organe de fermeture 42 de chaque ouverture 40, 41,
- un (ou plusieurs) capteur(s) chimique(s) 1, dit(s) par la suite capteur(s) à étalonner 1, et un (ou plusieurs) capteur(s) à photoionisation 2 positionnés dans le volume intérieur V de la chambre 4,
- un dispositif de pilotage 3 du système S pour la mise en oeuvre des étapes d'initialisation, de mesure et d'étalonnage, et
- dans certaines formes de réalisation de l'invention, un dispositif d'élimination du mélange gazeux 6 dans le volume intérieur V de la chambre 4.

On décrit par la suite successivement la chambre 4, les capteurs à étalonner 1 et à photoionisation 2, le dispositif d'élimination du mélange gazeux 6 et le dispositif de pilotage 3.

Dans l'exemple de la [Fig.2], la chambre 4 comprend deux ouvertures, à savoir une ouverture d'entrée 40 et une ouverture de sortie 41, permettant l'admission du mélange gazeux 6 dans le volume intérieur V ainsi que son refoulement. On précise que chaque ouverture 40, 41 permet la circulation du mélange gazeux 6 à identifier, tel que des polluants, mais également de l'air ambiant dans lequel il se trouve, tel que l'air ambiant d'un bureau dans lequel la chambre est montée. A noter que l'ouverture d'entrée 40 et l'ouverture de sortie 41 sont de préférence positionnées en vis-à-vis pour favoriser la circulation du mélange gazeux 6 dans tout le volume intérieur V de la chambre 4. Les ouvertures 40, 41 contribuent ainsi à ce que le mélange gazeux 6 au contact du capteur à étalonner 1 et du capteur à photoionisation 2 soit représentatif du mélange gazeux 6 dans tout le volume intérieur V, ce qui permet une mesure pertinente et une identification fiable. Il va de soi que la chambre 4 pourrait comprendre un nombre différent d'ouvertures, notamment une unique ouverture afin de permettre l'entrée et la sortie du mélange gazeux 6 afin de limiter l'encombrement.

Toujours en référence à la [Fig.2], pour améliorer la circulation et le renouvellement du mélange gazeux 6 dans le volume intérieur V de la chambre 4, le système S comprend dans cet exemple un organe de ventilation 5 monté dans la chambre 4, de préférence au niveau de l'ouverture de sortie 41. A titre d'exemple, l'organe de ventilation 5 se présente sous la forme d'un ventilateur ou d'une pompe aspirante. Il va de soi que l'organe de ventilation 5 pourrait ne pas être présent ou être monté différemment dans certaines formes de réalisation de l'invention, notamment pour limiter l'encombrement.

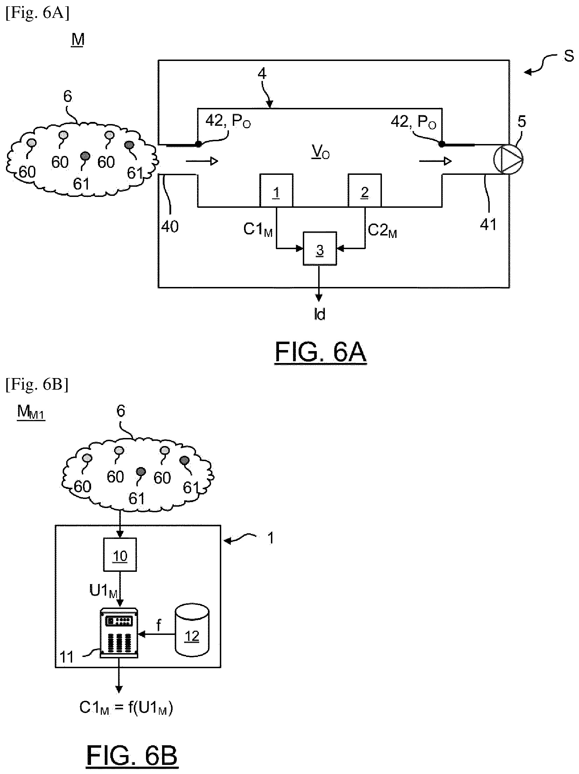

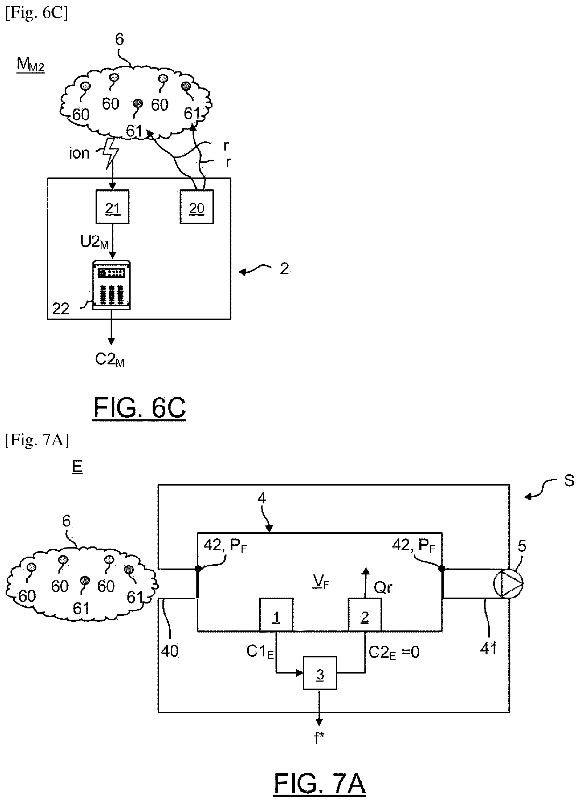

Selon l'invention et toujours en référence à la [Fig.2], chaque organe de fermeture 42 est monté mobile entre une position fermée P_{F} (adaptée pour les étapes d'initialisation et d'étalonnage) et une position ouverte P_{O} (adaptée pour l'étape de mesure). Dans la position fermée P_{F}, la chambre 4 délimite un volume intérieur fermé V_{F} (voir [Fig.7A] ), de préférence de manière étanche pour éviter toute admission fluidique extérieure à la chambre 4 et tout refoulement fluidique hors de la chambre 4. Dans la position ouverte P_{O}, la chambre 4 délimite un volume intérieur ouvert V_{O} (voir [Fig.6A]), c'est-à-dire permettant une admission fluidique dans la chambre 4, notamment de mélange gazeux 6, et un refoulement fluidique hors de la chambre 4. Dans l'exemple de la [Fig.2], chaque organe de fermeture 42 se présente sous la forme d'un clapet, monté pivotant de manière à ce qu'en position fermée P_{F}, le clapet obstrue l'ouverture 40, 41 et qu'en position ouverte P_{O}, le volume intérieur V est en communication fluidique avec le mélange gazeux 6. Il va de soi que l'organe de fermeture 42 pourrait être déplacé selon d'autres cinématiques, notamment une translation. L'organe de fermeture 42 pourrait également se présenter sous une autre forme, notamment celle d'une vanne.

Toujours en référence à la [Fig.2], la chambre 4 présente de préférence des dimensions réduites, légèrement supérieures à celles du capteur à étalonner 1 et du capteur à photoionisation 2. De préférence, la chambre 4 présente un volume inférieur à 10cm³. Ceci facilite l'action du dispositif d'élimination du mélange gazeux 6 dans le volume intérieur V comme ce sera décrit par la suite. Une telle chambre 4, et donc le système S, présente en outre un encombrement faible, ce qui permet son installation de manière simple et pratique dans tout type d'environnement.

Selon l'invention et en référence à la [Fig.3], de manière connue, le capteur à étalonner 1 comprend :
- un élément de mesure 10 configuré pour émettre un signal électrique U1 qui est fonction de la quantité d'un (ou plusieurs) composé(s) chimique(s) prédéterminé(s) dans le volume intérieur V et
- un élément de calcul 11 d'un premier signal physique C1 dudit composé chimique à partir dudit signal électrique U1 et d'une fonction de conversion f propre audit capteur à étalonner 1, stockée dans cet exemple dans une base de données 12, suivant la relation suivante : C1 = f(U1).

En pratique, le capteur à étalonner 1 est choisi sensible à minima au mélange gazeux 6 à mesurer, et plus précisément à au moins un composé chimique prédéterminé 60, 61 du mélange gazeux 6 (voir [Fig.2]), ainsi qu'à l'ozone, comme ce sera expliqué par la suite. Le capteur à étalonner 1 est ainsi configuré pour émettre un signal physique C1, telle qu'une concentration à titre d'exemple, du mélange gazeux 6 et de l'ozone présents dans le volume intérieur V de la chambre 4. Par ailleurs, le capteur à étalonner 1 est, comme son nom le suggère, sujet à la dérive, c'est-à-dire que pour un mélange gazeux 6 identique, l'élément de mesure 10 du capteur à étalonner 1 émet un signal électrique U1 différent au fur et à mesure de son utilisation. Autrement dit, la fonction de conversion f n'est plus représentative de la relation reliant le signal électrique U1 et le signal physique C1 correspondant.

De préférence, le capteur à étalonner 1 se présente sous la forme d'un capteur non sélectif, c'est-à-dire sensible à une pluralité de composés chimiques, afin que le système S soit adapté pour mesurer un large panel de mélanges gazeux 6. Un tel système S permet en outre une identification précise du mélange gazeux 6, grâce au capteur à photoionisation 2 qui est notamment configuré pour mesurer un deuxième signal physique du mélange gazeux 6, en complément du premier signal physique C1, comme ce sera décrit par la suite.

A titre d'exemple, le capteur à étalonner 1 se présente sous la forme d'un capteur semi-conducteur, également nommé « capteur MOX » ou « capteur MOS », pour lequel l'élément de mesure 10 comprend une couche d'oxyde métallique chauffée par un élément chauffant, sur laquelle viennent se fixer les composants chimique 60, 61 du mélange gazeux 6, ce phénomène étant connu sous le terme « d'adsorption ». L'élément de mesure 10 comprend en outre des électrodes de mesure configurées pour mesurer un signal électrique U1 correspondant à une conductivité électrique de la couche d'oxyde métallique. Pour un tel capteur, la dérive est notamment générée par l'obstruction progressive des pores de la couche d'oxyde métallique, qui modifie sa conductivité électrique. Alternativement, le capteur à étalonner 1 se présente sous la forme d'un capteur électrochimique. De tels capteurs sont connus en soi de l'homme du métier et ne seront pas décrits davantage.

Selon un aspect préféré, le capteur à étalonner 1 est choisi sensible aux composés organiques volatils, d'abréviation « COV », dans l'air. Par COV, on désigne à titre d'exemples les polluants émis par les moyens de transport, les imprimantes, les radiateurs, le tabagisme, la combustion de bougies ou encore les émissions émises par le mûrissement des fruits et légumes, et notamment le formaldéhyde, l'éthanol, l'acétone, l'acétaldéhyde, le benzène, le toluène et le xylène. Autrement dit, le mélange gazeux 6 à identifier se présente sous la forme de COV. Le capteur à étalonner 1 se présente ainsi sous la forme d'un capteur non sélectif à un unique composé chimique.

Selon l'invention et en référence à la [Fig.4], de manière connue, le capteur à photoionisation 2 comprend :
- un émetteur 20 de rayons ultraviolets r dans le volume intérieur V, tel qu'une source lumineuse à titre d'exemple, configuré pour photoioniser un (ou plusieurs) composé(s) chimique(s) prédéterminé(s) dans le volume intérieur V,
- un élément de mesure 21, tel que des électrodes de mesure par exemple, configuré pour émettre un signal électrique U2 qui est fonction de la quantité du composé chimique photoionisé et
- un élément de calcul 22 d'un deuxième signal physique C2 dudit composé chimique photoionisé à partir dudit signal électrique U2.

En pratique, le capteur à photoionisation 2 est configuré pour émettre un deuxième signal physique C2 du mélange gazeux 6, telle qu'une concentration de COV par exemple, pour compléter le premier signal physique C1 mesuré par le capteur à étalonner 1. La puissance d'émission de l'émetteur 20 de rayons ultraviolets r est ainsi choisie suffisamment grande pour photoioniser le mélange gazeux 6 et suffisamment faible pour que le capteur à photoionisation 2 soit sélectif. Outre sa fonction de capteur de mesure, le capteur à photoionisation 2 est également configuré suivant une deuxième fonction (adaptée pour l'étape d'étalonnage) pour générer de l'ozone dans le volume intérieur V. Plus précisément, l'émetteur 20 est configuré pour fournir une puissance d'émission de rayons ultraviolets r de telle sorte à photoioniser le dioxygène naturellement présent en ozone. La puissance d'émission de rayons ultraviolets r adaptée pour l'étalonnage est de préférence supérieure à celle adaptée pour la mesure.

A noter que dans l'exemple de la [Fig.2], le système S comprend un unique capteur à étalonner 1 et un unique capteur à photoionisation 2 pour limiter l'encombrement et le coût. Il va cependant de soi que le système S pourrait comprendre une pluralité de capteurs à étalonner 1, en particulier sensibles à différents mélanges gazeux 6 pour augmenter le panel de mesure du système S. Le système S pourrait également comprendre plusieurs capteurs à photoionisation 2, notamment, pour que chaque capteur à étalonner 1 soit associé à un capteur à photoionisation 2.

Comme décrit précédemment, le système S comprend dans certaines formes de réalisation un dispositif d'élimination du mélange gazeux 6 se présentant, dans l'exemple de la [Fig.2], sous la forme du capteur à photoionisation 2. En effet, le capteur à photoionisation 2, et plus précisément l'émetteur 20 de rayons ultraviolets r, permet également suivant une troisième fonction d'éliminer le mélange gazeux 6 dans le volume intérieur V et ce de deux façons combinées :
- d'une première façon « directe », en photoionisant le mélange gazeux 6, à savoir en le transformant en ions par définition, et
- d'une deuxième façon « indirecte », en générant de l'ozone O par photoionisation du dioxygène D naturellement présent dans l'air, qui réagit par ozonolyse avec le mélange gazeux 6.

En pratique, l'ozonolyse permet d'éliminer la majeure partie du mélange gazeux 6 présent dans le volume intérieur V, à savoir environ 90% du mélange gazeux 6 présent, et la photoionisation la partie restante, ce qui permet une élimination efficace et rapide. Ainsi, le capteur à photoionisation 2 est ainsi avantageusement configuré pour remplir trois fonctions, à savoir celle de mesure du mélange gazeux 6, celle de générateur d'ozone et celle d'élimination du mélange gazeux 6, ce qui réduit avantageusement l'encombrement et le coût.

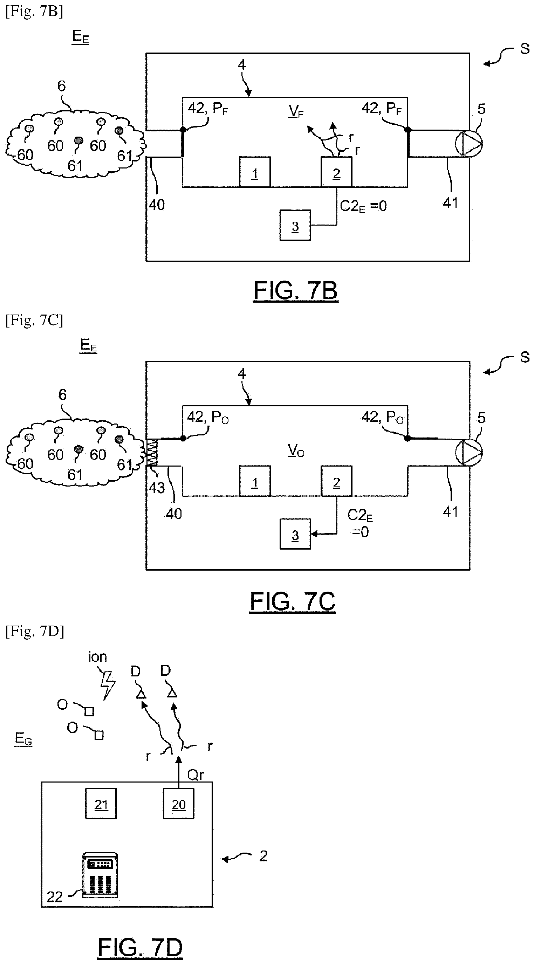

De manière alternative, le dispositif d'élimination du mélange gazeux 6 se présente sous la forme de l'organe de ventilation 5 conjointement avec un organe de filtrage 43 (voir [Fig.7C]). L'organe de ventilation 5 est dans cette alternative de préférence monté au niveau de l'ouverture de sortie 41 et est configuré pour évacuer le mélange gazeux 6 à l'extérieur de la chambre 4. L'organe de filtrage 43 est quant à lui monté mobile au niveau de l'ouverture d'entrée 40 entre une position déployée, dans laquelle il est configuré pour filtrer le mélange gazeux 6 admis dans le volume intérieur V, et une position escamotée, dans laquelle le mélange gazeux 6 pénètre librement dans le volume intérieur V de la chambre 4, c'est-à-dire, sans filtrage. La nature de l'organe de filtrage 43 dépend du mélange gazeux 6 à mesurer. A titre d'exemple, l'organe de filtrage 43 se présente sous la forme d'un filtre chimique configuré pour réagir avec le mélange gazeux 6 ou d'un filtre physique configuré pour adsorber le mélange gazeux 6. De tels organes de filtrage 43 sont connus en soi de l'homme du métier et ne seront pas décrits davantage. Un tel dispositif d'élimination du mélange gazeux est avantageusement peu complexe et efficace, empêchant au niveau de l'ouverture d'entrée 40 l'admission du mélange gazeux 6 et favorisant au niveau de l'ouverture de sortie 41 l'évacuation du mélange gazeux 6 éventuellement rémanent du volume intérieur V.

De manière alternative, le système S est exempt de dispositif d'élimination du mélange gazeux 6, l'étalonnage étant mis en oeuvre à un moment opportun où le mélange gazeux 6 n'est pas présent.

Toujours en référence à la [Fig.2], le dispositif de pilotage 3 se présente de préférence sous la forme d'un calculateur informatique qui comprend, de préférence, une mémoire. Le dispositif de pilotage 3 est configuré pour piloter le déplacement des organes de fermeture 42, et suivant certaines formes de réalisation le déplacement de l'organe de filtrage 43. Le dispositif de pilotage 3 est également configuré pour identifier le mélange gazeux 6 à partir des mesures des capteurs à étalonner 1 et à photoionisation 2 lors d'une étape de mesure, et lors d'une étape d'étalonnage, pour déterminer une fonction de conversion f optimisée pour le capteur à étalonner 1, comme ce sera expliqué par la suite.

Pour résumer, le système S de l'invention comprend un capteur à étalonner 1 et un capteur à photoionisation 2 positionnés dans une chambre 4, délimitant un volume ouvert ou fermé, exempt ou non d'un mélange gazeux 6 à mesurer et commandée par un dispositif de pilotage 3. Pour un gain en coût, en temps et en encombrement, le capteur à photoionisation 2 est multifonctionnel : il permet l'identification du mélange gazeux 6 en coopération avec le capteur à étalonner 1 et, lorsque le capteur 1 doit être étalonné, il forme un générateur d'ozone pour créer un milieu étalon dans la chambre 4.

On décrit par la suite le procédé d'utilisation du système S, qui permet suivant une première utilisation l'identification d'un mélange gazeux 6, ce qui correspond sur la [Fig.5] à une étape de mesure M, et suivant une deuxième utilisation l'étalonnage du capteur à étalonner 1, ce qui correspond sur la même [Fig.5] à une étape d'étalonnage E.

En référence à la [Fig.5], l'étape de mesure M et l'étape d'étalonnage E sont mises en oeuvre consécutivement et non simultanément. En pratique, lors de l'utilisation du système S, l'étape de mesure M est mise en oeuvre jusqu'à ce qu'un étalonnage soit requis pour le capteur à étalonner 1. A titre d'exemple, un étalonnage est requis après le constat d'une identification erronée ou imprécise du mélange gazeux 6 ou après une période d'utilisation prédéterminée à partir de laquelle le capteur à étalonner 1 est susceptible de dériver. L'étape de mesure M est alors stoppée pour débuter l'étape d'étalonnage E. Une fois l'étape d'étalonnage E terminée, c'est-à-dire une fois que le capteur 1 est étalonné, une nouvelle étape de mesure M est mise en oeuvre. En pratique, le procédé d'utilisation du système S comporte ainsi une alternance d'étapes de mesure M et d'étapes d'étalonnage E.

Dans l'exemple de la [Fig.5], le procédé d'utilisation du système S comporte de plus une étape d'initialisation 1 préliminaire, qui comme ce sera décrit par la suite augmente la précision et la fiabilité de (ou des) étape(s) d'étalonnage E.

On décrit par la suite successivement une étape de mesure M, une étape d'étalonnage E et une étape d'initialisation I.

Selon l'invention et en référence aux figures 5 et 6A, lors d'une étape de mesure M :
- le dispositif de pilotage 3 commande le déplacement M_{D} des organes de fermeture 42 en position ouverte P_{O} de manière à ce que la chambre 4 délimite un volume intérieur ouvert V_{O} dans lequel pénètre le mélange gazeux 6,
- le capteur à étalonner 1 et le capteur à phototoionisation 2 mesurent M_{M1}, M_{M2} respectivement un premier signal physique de mesure C1_{M} et un deuxième signal physique de mesure C2_{M} du mélange gazeux 6, et
- le dispositif de pilotage 3 identifie M_{S} le mélange gazeux 6 à partir des signaux physiques de mesure C1_{M}, C2_{M},

Lorsque le volume intérieur est ouvert V_{O}, le mélange gazeux 6 circule au contact du capteur à étalonner 1 et du capteur à photoionisation 2, ce qui permet une mesure précise et fiable de la concentration du mélange gazeux 6 présent dans toute la chambre 4 et non localement. Dans l'exemple de la [Fig.6A], le dispositif de pilotage 3 commande également l'activation de l'organe de ventilation 5 pour favoriser la circulation et le renouvellement du mélange gazeux 6 dans la chambre 4. On précise que chaque organe de fermeture 42 en position ouverte P_{O} laisse circuler dans la chambre 4 le mélange gazeux 6 à identifier mais également l'air ambiant dans lequel le mélange gazeux 6 se trouve. A titre d'exemple, la chambre 4 est positionnée dans un bureau où l'occupation humaine génère des polluants dans l'air ambiant et est configurée pour admettre dans le volume intérieur ouvert V_{O} l'air ambiant chargé en polluants, les polluants formant le mélange gazeux 6 à identifier.

De préférence, les deux signaux physiques de mesure C1_{M}, C2_{M} sont mesurés simultanément de manière à correspondre au même mélange gazeux 6. Les signaux physiques de mesure C1_{M}, C2_{M} correspondent par ailleurs de préférence à des mesures moyennées pour augmenter leur fiabilité et leur précision, mais il va de soi qu'il peut également s'agir de mesures instantanées. En référence aux figures 6B et 6C, les signaux physiques de mesure C1_{M}, C2_{M} sont respectivement obtenus à partir de signaux électriques U1_{M}, U2_{M}. De telles étapes de mesure individuelles sont connues de l'homme du métier.

En référence à la [Fig.5], l'identification M_{S} du mélange gazeux 6 est mise en oeuvre par comparaison des signaux physiques de mesure C1_{M}, C2_{M} avec une base de données de référence. L'identification est avantageusement fiable et précise, car le deuxième signal physique de mesure C2_{M} permet de vérifier la cohérence et/ou de compléter le premier signal physique de mesure C1_{M}. Autrement dit, le capteur à photoionisation 2 est réputé non sujet à la dérive et permet un contrôle de la mesure du capteur à étalonner 1. Une incohérence entre les deux signaux physiques de mesure C1_{M}, C2_{M} peut notamment révéler une dérive du capteur à étalonner 1 et donc nécessiter la mise en oeuvre d'une étape d'étalonnage E.

Selon l'invention et en référence aux figures 5 et 7A, lors d'une étape d'étalonnage E :
- le dispositif de pilotage 3 commande le déplacement E_{D} des organes de fermeture 42 en position fermée P_{F} de manière à ce que la chambre 4 délimite un volume intérieur fermé V_{F},
- lorsque le volume intérieur fermé V_{F} est exempt de mélange gazeux 6 et d'ozone O, le capteur à photoionisation 2 génère E_{G} de l'ozone O dans le volume intérieur fermé V_{F} en émettant suivant une puissance donnée Qr des rayons ultraviolets r configurés pour photoioniser le dioxygène D présent,
- le capteur à étalonner 1 mesure E_{M} un signal physique d'étalonnage C1_{E} de l'ozone O généré,
- le dispositif de pilotage 3 calcule E_{C} l'écart ε entre le signal physique d'étalonnage C1_{E} et un signal physique de référence C1_{I} et
- si l'écart ε est supérieur à un écart de référence εref, le dispositif de pilotage 3 détermine E_{S} une fonction de conversion optimisée f* pour le capteur à étalonner 1 à partir dudit écart ε, de manière à l'étalonner.

On précise que le dioxygène D naturellement présent dans la chambre 4 provient de l'air ambiant admis au même titre que le mélange gazeux 6, en pratique avant le déplacement des organes de fermeture 42 en position fermée P_{F}.

Dans les formes de réalisation illustrées sur les figures 7B et 7C où le système S comprend en outre un dispositif d'élimination du mélange gazeux 6, l'étape d'étalonnage comprend de plus, avant la génération E_{G} d'ozone O, une élimination E_{E} du mélange gazeux 6 éventuellement rémanent dans le volume intérieur V.

En pratique, le déplacement E_{D} des organes de fermeture 42 est mis en oeuvre avant ou après l'élimination E_{E} du mélange gazeux 6 suivant les modes de réalisation de l'invention. Dans l'exemple de la [Fig.7B] où le capteur à photoionisation 2 forme le dispositif d'élimination du mélange gazeux 6, le déplacement E_{D} des organes de fermeture 42 est mis en oeuvre en premier, de manière à ce que la chambre 4 délimite un volume intérieur fermé V_{F} étanche. Le capteur à photoionisation 2 émet ensuite des rayons ultraviolets r dans le volume intérieur fermé V_{F} afin de photoioniser tout le mélange gazeux 6 éventuellement présent.

Dans l'exemple de la [Fig.7C] où l'organe de filtrage 43 et l'organe de ventilation 5 forment conjointement le dispositif d'élimination du mélange gazeux 6, l'élimination E _{E} du mélange gazeux 6 est mise en oeuvre dans le volume intérieur ouvert V_{O}. Pour cela, le dispositif de pilotage 3 déplace l'organe de filtrage 43 en position déployée de manière à empêcher l'admission du mélange gazeux 6 au niveau de l'ouverture d'entrée 40. Le dispositif de pilotage 3 commande également l'activation de l'organe de ventilation 5 afin d'évacuer le mélange gazeux 6 éventuellement rémanent dans le volume intérieur V à l'extérieur de la chambre 4. Une fois le mélange gazeux 6 éliminé E_{E} dans la chambre 4, le dispositif de pilotage 3 déplace les organes de fermeture 42 en position fermée P_{F}.

De préférence et comme illustré sur les figures 7A à 7C, l'élimination E_{E} du mélange gazeux 6 est contrôlée par le capteur à photoionisation 2 par la mesure d'un signal physique de contrôle C2_{E} du mélange gazeux 6 dans le volume intérieur. Si le signal physique de contrôle C2_{E} correspond à une concentration sensiblement nulle de mélange gazeux 6, l'étape d'étalonnage E est poursuivie. Autrement, l'élimination E_{E} est réitérée jusqu'à ce que la chambre 4 soit exempte de mélange gazeux 6. On précise que l'élimination E_{E} ne permet pas de faire le vide dans la chambre 4 mais uniquement de retirer le mélange gazeux 6 à identifier, l'air ambiant étant toujours présent.

Alternativement, l'étape d'étalonnage E est exempte d'élimination E_{E} du mélange gazeux 6 et est mise en oeuvre à un moment opportun, lorsque le mélange gazeux 6 à identifier n'est pas présent dans le volume intérieur V. Dans l'exemple d'une chambre 4 montée dans un bureau dont l'occupation génère des polluants dans l'air ambiant, l'étape d'étalonnage E est ainsi de préférence mise en oeuvre lorsque l'occupation est faible ou nulle. Autrement dit, l'étape d'étalonnage E est préférentiellement mise en oeuvre lorsque l'air ambiant admis dans la chambre 4 est faiblement chargé ou non chargé en mélange gazeux 6 à identifier. Un tel moment opportun est déterminé à titre d'exemples grâce à l'historique des mesures du capteur à étalonner 1 et du capteur à photoionisation 2.

En référence à la [Fig.7D], la génération E_{G} d'ozone O est mise en oeuvre dans un volume intérieur fermé V_{F} via une émission de rayons ultraviolets r suivant une puissance donnée Qr choisie. La génération E_{G} d'ozone O permet ainsi de créer un milieu étalon connu dans la chambre 4, c'est-à-dire de concentration d'ozone O connue, via la puissance d'émission Qr, et de concentration en mélange gazeux 6 connue, à savoir sensiblement nulle. En pratique, pour que le volume intérieur fermé V _{F} forme un milieu étalon connu, celui-ci doit, avant la mise en oeuvre de la génération E _{G} d'ozone O, être exempt de mélange gazeux 6 et d'ozone O. Pour le mélange gazeux 6, ceci est opéré par l'élimination E_{E} préliminaire ou le choix d'un moment opportun pour l'étalonnage. La génération E_{G} d'ozone O est de plus opérée de préférence après un temps d'attente pour que l'ozone O éventuellement rémanent dans le volume intérieur fermé V_{F} se décompose spontanément au contact des parois de la chambre 4.

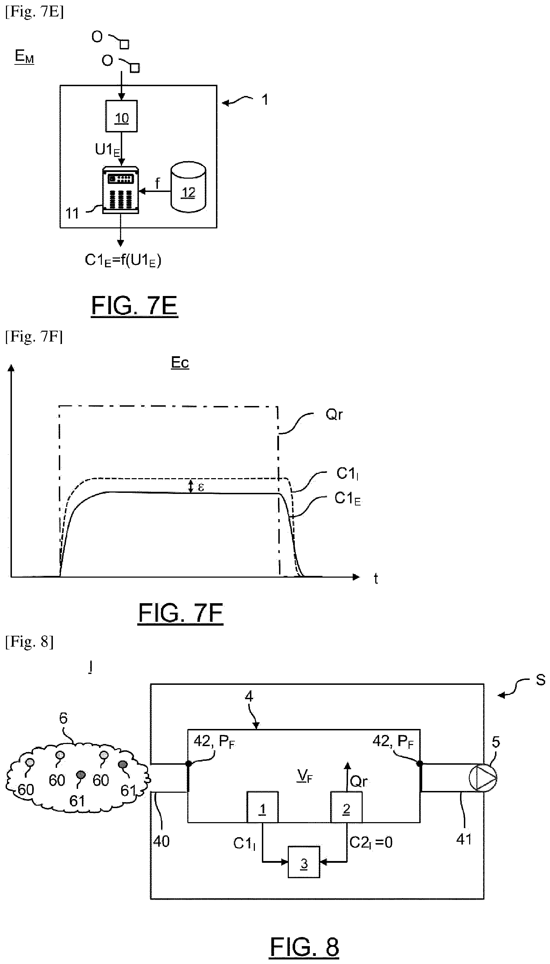

En référence à la [Fig.7E], le signal physique d'étalonnage C1_{E} mesuré par le capteur à étalonner 1 dans un tel milieu étalon correspond uniquement à l'ozone O présent dans le volume intérieur fermé V_{F} et dont la concentration est connue. En référence à la [Fig.7F], le dispositif de pilotage 3 peut ainsi calculer la potentielle dérive du capteur à étalonner 1 en comparant le signal physique d'étalonnage C1_{E} avec un signal physique de référence C1_{I} de l'ozone O présent dans la même concentration. Dans l'exemple de la [Fig.7F], l'écart ε est obtenu de la manière suivante : ε = |C1_{E}-C1_{I}|/C1_{I}. L'écart de référence εref est de préférence choisi compris entre 1% et 10%, préférentiellement de l'ordre de 5%, afin de corriger toute dérive significative du capteur à étalonner 1. Dans le cas d'une dérive, la fonction de conversion optimisée f* est calculée de la manière suivante : f*(U1_{E}) = C1_{I}.

De préférence, le signal physique de référence C1_{I} est déterminé lors de l'étape d'initialisation 1 préliminaire du procédé d'utilisation du système S. En référence à la [Fig.8], lors d'une telle étape d'initialisation I, le capteur à étalonner 1 est réputé valide et :
- le dispositif de pilotage 3 commande le déplacement I_{D} de l'organe de fermeture 42 en position fermée P_{F} de manière à ce que la chambre 4 délimite un volume intérieur fermé V_{F},
- lorsque le volume intérieur fermé V_{F} est exempt de mélange gazeux 6 et d'ozone O, le capteur à photoionisation 2 génère I_{G} de l'ozone O dans le volume intérieur fermé V_{F} en émettant suivant ladite puissance donnée Qr des rayons ultraviolets r configurés pour photoioniser le dioxygène D présent et
- le capteur à étalonner 1 mesure I_{M} ledit signal physique de référence C1_{I} de l'ozone O généré.

De manière analogue à l'étape d'étalonnage E, dans les formes de réalisation illustrées sur les figures 7B et 7C où le système S comprend en outre un dispositif d'élimination du mélange gazeux 6, l'étape d'initialisation 1 comprend de plus, avant la génération I_{G} d'ozone O, une élimination I_{E} du mélange gazeux 6 éventuellement rémanent dans le volume intérieur V.

On notera les similarités entre l'étape d'initialisation 1 et les quatre premières actions de l'étape d'étalonnage E. L'étape d'initialisation 1 ne sera ainsi pas plus décrite, chacune de ses actions étant décrite par l'action du même nom de l'étape d'étalonnage E à laquelle on se réfèrera. Une telle étape d'initialisation 1 permet avantageusement un étalonnage précis et fiable du capteur à étalonner 1. En effet, le signal physique de référence C1_{I} est mesuré dans le même milieu étalon que le signal physique d'étalonnage C1_{E} et avec le même moyen de mesure, à savoir le capteur à étalonner 1. Toutefois lors de l'étape d'initialisation I, le capteur à étalonner 1 est supposé étalonné contrairement à l'étape d'étalonnage E. L'étape d'initialisation 1 est ainsi de préférence mise en oeuvre avant la première étape de mesure M. Il va cependant de soi que le signal physique de référence C1_{I} peut être obtenu de manière différente, notamment grâce à une base de données.

On notera également que dans le cas d'une chambre 4 comprenant plusieurs capteurs à étalonner 1, un unique capteur à étalonner 1 est étalonné lors d'une étape d'étalonnage E et/ou lors d'une étape d'initialisation I. Ceci permet d'éviter de modifier le milieu étalon et de garantir la fiabilité et la précision de l'étalonnage. L'étape de mesure M peut quant à elle être mise en oeuvre par un ou plusieurs capteurs à étalonner 1 simultanément, pour augmenter la précision et la fiabilité de l'identification du mélange gazeux 6.

Pour résumer, lors d'une étape de mesure M au moyen du système S, le capteur à étalonner 1 et le capteur à photoionisation 2 mesurent chacun un signal physique de mesure C1_{M}, C2_{M} du mélange gazeux 6 dans le volume intérieur ouvert V_{O}, ce qui permet au dispositif de pilotage 3 de discriminer le mélange gazeux 6. Lorsqu'un étalonnage E est requis, la chambre 4 forme un milieu étalon connu dans lequel le capteur à étalonner 1 mesure un signal physique d'étalonnage C1_{E} qui, par comparaison avec un signal physique de référence C1_{I}, permet de corriger la potentielle dérive du capteur à étalonner 1. Le capteur à photoionisation 2, permet par ozonolyse et photoionisation du mélange gazeux 6 éventuellement rémanent dans la chambre 4 et du dioxygène présent, d'éliminer le mélange gazeux 6 et de générer de l'ozone O. Alternativement, l'élimination du mélange gazeux 6 est mise en oeuvre conjointement par un organe de filtrage 43 et un organe de ventilation 5.

Selon un aspect préféré de l'invention, les signaux physiques C1, C2 du capteur à étalonner 1 et du capteur à photoionisation 2 se présentent sous la forme de concentrations du mélange gazeux 6 dans la chambre 4. Autrement dit, le premier signal physique de mesure C1_{M} et le deuxième signal physique de mesure C2_{M} se présentent de préférence respectivement sous la forme d'une première concentration de mesure et d'une deuxième concentration de mesure du mélange gazeux 6 dans la chambre 4. Le signal physique d'étalonnage C1_{E} et le signal physique de référence C1_{I} de l'ozone O dans la chambre 4 se présentent de préférence respectivement sous la forme d'une concentration d'étalonnage d'ozone et d'une concentration d'ozone de référence.

## Revendications

1. Procédé d'utilisation d'un système de captation chimique à étalonnage intégré (S) pour la mesure d'un mélange gazeux (6), ledit système (S) comprenant :
• une chambre (4) délimitant un volume intérieur (V) et comprenant au moins une ouverture (40, 41) de mise en communication fluidique du mélange gazeux (6) et du volume intérieur (V) et au moins un organe de fermeture (42) de ladite ouverture (40, 41) monté mobile entre une position fermée (P_{F} ) délimitant un volume intérieur fermé (V_{F}) et une position ouverte (P_{O}) délimitant un volume intérieur ouvert (V_{O}),
• au moins un capteur chimique, dit capteur à étalonner (1), positionné dans le volume intérieur (V) de la chambre (4) et comprenant un élément de mesure (10) configuré pour émettre un signal électrique (U1) qui est fonction de la quantité d'au moins un composé chimique prédéterminé (60, 61, O) dans le volume intérieur (V), ledit capteur à étalonner (1) comprenant un élément de calcul (11) d'un premier signal physique (C1) dudit composé chimique (60, 61, O) à partir dudit signal électrique (U1) et d'une fonction de conversion (f) propre audit capteur à étalonner (1) suivant la relation suivante : C1 = f(U1),
• au moins un capteur à photoionisation (2) positionné dans le volume intérieur (V), réputé non sujet à la dérive, et configuré, d'une part, pour émettre des rayons ultraviolets (r) de manière à photoioniser au moins un composé chimique prédéterminé (61, D) dans le volume intérieur (V), et d'autre part, pour déterminer un deuxième signal physique (C2) qui est fonction de la quantité dudit composé chimique (61) photoionisé, et
• au moins un dispositif de pilotage (3),
• procédé dans lequel, lors d'au moins une étape de mesure (M)
• le dispositif de pilotage (3) commande le déplacement (M_{D}) de l'organe de fermeture (42) en position ouverte (P_{O}) de manière à ce que la chambre (4) délimite un volume intérieur ouvert (V_{O}) dans lequel pénètre le mélange gazeux (6),
• le capteur à étalonner (1) et le capteur à phototoionisation (2) mesurent (M_{M1}, M_{M2}) respectivement au moins un premier signal physique de mesure (C1_{M}) et au moins un deuxième signal physique de mesure (C2 _{M}) du mélange gazeux (6), et
• le dispositif de pilotage (3) identifie (M_{S}) le mélange gazeux (6) à partir des signaux physiques de mesure (C1_{M}, C2_{M}),
• procédé **caractérisé par le fait que** lors d'au moins une étape d'étalonnage (E) :
• le dispositif de pilotage (3) commande le déplacement (E_{D}) de l'organe de fermeture (42) en position fermée (P_{F}) de manière à ce que la chambre (4) délimite un volume intérieur fermé (V_{F}),
• lorsque le volume intérieur fermé (V_{F}) est exempt de mélange gazeux (6) et d'ozone (O), le capteur à photoionisation (2) génère (E_{G}) de l'ozone (O) dans le volume intérieur fermé (V_{F}) en émettant suivant une puissance donnée (Qr) des rayons ultraviolets (r) configurés pour photoioniser le dioxygène (D) présent,
• le capteur à étalonner (1) mesure (E_{M}) un signal physique d'étalonnage (C1_{E}) de l'ozone (O) généré,
• le dispositif de pilotage (3) calcule (E_{C}) l'écart (ε) entre le signal physique d'étalonnage (C1_{E}) et un signal physique de référence (C1_{I}), fonction de la puissance donnée (Qr) du capteur à photoionisation (2), et
• si l'écart (ε) est supérieur à un écart de référence (εref), le dispositif de pilotage (3) détermine (E_{S}) une fonction de conversion optimisée (f*) pour le capteur à étalonner (1) à partir dudit écart (ε), de manière à l'étalonner.

2. Procédé d'utilisation selon la revendication 1, dans lequel lors de l'étape d'étalonnage (E), le système (S) comprenant au moins un dispositif d'élimination du mélange gazeux (6) dans le volume intérieur (V), le dispositif d'élimination élimine (E_{E}) le mélange gazeux (6) éventuellement rémanent dans le volume intérieur (V) avant la génération (E _{G}) d'ozone (O).

3. Procédé d'utilisation selon la revendication 2, dans lequel, la chambre (4) comprenant au moins une ouverture d'entrée (40) et au moins une ouverture de sortie (41), le système de captation (S) comprenant au moins un organe de ventilation (5) monté dans la chambre (4), de préférence au niveau de l'ouverture de sortie (41), et au moins un organe de filtrage (43) du mélange gazeux (6) monté mobile au niveau de l'ouverture d'entrée (40) entre une position déployée et une position escamotée et formant avec l'organe de ventilation (5) le dispositif d'élimination du mélange gazeux (6), l'élimination (E_{E}) du mélange gazeux (6) lors de l'étape d'étalonnage (E_{E}) est mise en oeuvre dans le volume intérieur ouvert (V_{O}) par le dispositif de pilotage (3) en commandant conjointement le déplacement de l'organe de filtrage (43) en position déployée (P_{D}) et l'activation de l'organe de ventilation (5), de manière à éviter l'admission de mélange gazeux (6) dans le volume intérieur ouvert (V_{O}) et à évacuer le mélange gazeux (6) éventuellement rémanent hors du volume intérieur ouvert (V_{O}).

4. Procédé d'utilisation selon la revendication 2, dans lequel, le capteur à photoionisation (2) formant le dispositif d'élimination du mélange gazeux (6), l'élimination (E_{E}) du mélange gazeux (6) lors de l'étape d'étalonnage (E_{E}) est mise en oeuvre dans le volume intérieur fermé (V_{F}) par le capteur à photoionisation (2) en émettant des rayons ultraviolets (r) configurés, d'une part, pour générer de l'ozone (O) par photoionisation du dioxygène (D) présent afin de réagir par ozonolyse avec le mélange gazeux (6) éventuellement rémanent dans le volume intérieur fermé (V_{F}), et d'autre part pour photoioniser ledit mélange gazeux (6).

5. Procédé d'utilisation selon l'une des revendications 1 à 4, dans lequel, lors de l'étape de mesure (M), le premier signal physique de mesure (C1 _{M}) et le deuxième signal physique de mesure (C2_{M}) sont mesurés simultanément.

6. Procédé d'utilisation selon l'une des revendications 1 à 5, dans lequel, lors d'au moins une étape d'initialisation (1) :
• le dispositif de pilotage (3) commande le déplacement (I_{D}) de l'organe de fermeture (42) en position fermée (P_{F}) de manière à ce que la chambre (4) délimite un volume intérieur fermé (V_{F} ),
• lorsque le volume intérieur fermé (V_{F}) est exempt de mélange gazeux (6) et d'ozone (O), le capteur à photoionisation (2) génère (I_{G}) de l'ozone (O) dans le volume intérieur fermé (V_{F}) en émettant suivant ladite puissance donnée (Qr) des rayons ultraviolets (r) configurés pour photoioniser le dioxygène (D) présent et
• le capteur à étalonner (1) mesure (I_{M}) ledit signal physique de référence (C1_{I}) de l'ozone (O) généré.

7. Système de captation chimique à étalonnage intégré (S) pour la mesure d'un mélange gazeux (6), ledit système (S) comprenant :
• une chambre (4) délimitant un volume intérieur (V) et comprenant au moins une ouverture (40, 41) de mise en communication fluidique du mélange gazeux (6) et du volume intérieur (V) et au moins un organe de fermeture (42) de ladite ouverture (40, 41) monté mobile entre une position fermée (P_{F} ) délimitant un volume intérieur fermé (V_{F}) et une position ouverte (P_{O}) délimitant un volume intérieur ouvert (V_{O}),
• au moins un capteur chimique, dit capteur à étalonner (1), positionné dans le volume intérieur (V) de la chambre (4) et comprenant un élément de mesure (10) configuré pour émettre un signal électrique (U1) qui est fonction de la quantité d'au moins un composé chimique prédéterminé (60, 61, O) dans le volume intérieur (V), ledit capteur à étalonner (1) comprenant un élément de calcul (11) d'un premier signal physique (C1) dudit composé chimique (60, 61, O) à partir dudit signal électrique (U1) et d'une fonction de conversion (f) propre audit capteur à étalonner (1) suivant la relation suivante : C1 = f(U1), ledit capteur à étalonner (1) étant en particulier configuré pour déterminer, respectivement lors d'une étape de mesure (M) et lors d'une étape d'étalonnage (E) du procédé d'utilisation selon l'une des revendications 1 à 6, un premier signal physique de mesure (C1_{M}) du mélange gazeux (6) dans le volume intérieur ouvert (V_{O}) et un signal physique d'étalonnage (C1_{E}) de l'ozone (O) dans le volume intérieur fermé (V_{F}),
• au moins un capteur à photoionisation (2) positionné dans le volume intérieur (V) et configuré, d'une part, pour émettre des rayons ultraviolets (r) de manière à photoioniser au moins un composé chimique prédéterminé (61, D) dans le volume intérieur (V), et d'autre part, pour déterminer un deuxième signal physique (C2) qui est fonction de la quantité dudit composé chimique (61) photoionisé, ledit capteur à photoionisation (2) étant en particulier configuré, lors de l'étape de mesure (M), pour déterminer un deuxième signal physique de mesure (C2_{M}) du mélange gazeux (6) dans le volume intérieur ouvert (V_{O}) et, lors de l'étape d'étalonnage (E), pour émettre des rayons ultraviolets (r) suivant une puissance donnée (Qᵣ) pour photoioniser le dioxygène (D) en ozone (O) dans le volume intérieur fermé (V_{F}), et
• au moins un dispositif de pilotage (3) configuré pour commander le déplacement de l'organe de fermeture (42) en position ouverte (P_{O}) lors de l'étape de mesure (M), de manière à ce que la chambre (4) délimite un volume intérieur ouvert (V_{O}), et en position fermée (P_{F}) lors de l'étape d'étalonnage (E), de manière à ce que la chambre (4) délimite un volume intérieur fermé (V_{F}), ledit dispositif de pilotage (3) étant configuré :
• lors de l'étape de mesure (M), pour identifier le mélange gazeux (6) à partir du premier et du deuxième signal physique de mesure (C1_{M}, C2_{M}) et,
• lors de l'étape d'étalonnage (E), pour calculer l'écart (ε) entre le signal physique d'étalonnage (C1_{E}) et un signal physique de référence (C1_{I}), fonction de la puissance donnée (Qr) du capteur à photoionisation (2), et, si l'écart (ε) est supérieur à un écart de référence (εref), déterminer une fonction de conversion optimisée (f*) pour le capteur à étalonner (1), de manière à l'étalonner.

8. Système de captation chimique (S) selon la revendication 7, dans lequel la chambre (4) comprend au moins une ouverture d'entrée (40), au moins une ouverture de sortie (41) et au moins un organe de fermeture (42) de l'ouverture d'entrée (40) et de l'ouverture de sortie (41), afin de faciliter la circulation du mélange gazeux (6) dans le volume intérieur ouvert (V_{O}).

9. Système de captation chimique (S) selon l'une des revendications 7 et 8, comprenant au moins un organe de ventilation (5) monté dans la chambre (4), de préférence au niveau de l'ouverture de sortie (41), pour favoriser le renouvellement du mélange gazeux (6) dans le volume intérieur ouvert (V_{O}).

10. Système de captation chimique (S) selon l'une des revendications 7 à 9, dans lequel le capteur à étalonner (1) se présente sous la forme d'un capteur semi-conducteur ou d'un capteur électrochimique.

## Patentansprüche

1. Verfahren für die Anwendung eines chemischen Erfassungssystems mit integrierter Kalibrierung (S) zum Messen eines Gasgemischs (6), wobei das System (S) umfasst:
• eine Kammer (4), die ein Innenvolumen (V) begrenzt und mindestens eine Öffnung (40, 41) zum Versetzen des Gasgemischs (6) und des Innenvolumens (V) in Fluidkommunikation und mindestens eine Verschlusseinrichtung (42) der Öffnung (40, 41) umfasst, die zwischen einer geschlossenen Position (P_{F}), die ein geschlossenes Innenvolumen (V_{F}) begrenzt, und einer geöffneten Position (Po), die ein offenes Innenvolumen (Vo) begrenzt, beweglich angebracht ist,
• mindestens einen chemischen Sensor, bezeichnet als zu kalibrierender Sensor (1), der in dem Innenvolumen (V) der Kammer (4) positioniert ist und ein Messelement (10) umfasst, das konfiguriert ist, um ein elektrisches Signal (U1) zu senden, das von der Menge mindestens einer vorbestimmten chemischen Verbindung (60, 61, O) in dem Innenvolumen (V) abhängt, wobei der zu kalibrierende Sensor (1) ein Berechnungselement (11) eines ersten physikalischen Signals (C1) der chemischen Verbindung (60, 61, O) auf der Basis des elektrischen Signals (U1) und einer eigenen Konvertierungsfunktion (f) des zu kalibrierenden Sensors (1) umfasst gemäß der folgenden Gleichung: C1 = f(U1),
• mindestens einen Photoionisationssensor (2), der in dem Innenvolumen (V) positioniert ist, der als nicht driftend gilt, und zum einen konfiguriert ist, um ultraviolette Strahlen (r) derart zu senden, dass mindestens eine vorbestimmte chemische Verbindung (61, D) in dem Innenvolumen (V) photoionisiert wird und zum anderen, um ein zweites physikalisches Signal (C2) zu bestimmen, das von der Menge der photoionisierten chemischen Verbindung (61) abhängt, und
• mindestens eine Steuervorrichtung (3),
wobei in dem Verfahren bei mindestens einem Messschritt (M):
• die Steuervorrichtung (3) die Verlagerung (M_{D}) der Verschlusseinrichtung (42) in die geöffnete Position (P_{O}) derart steuert, dass die Kammer (4) ein offenes Innenvolumen (Vo) begrenzt, in welches das Gasgemisch (6) eindringt,
• der zu kalibrierende Sensor (1) und der Photoionisationssensor (2) jeweils mindestens ein erstes physikalisches Messsignal (C1_{M}) und mindestens ein zweites physikalisches Messsignal (C2_{M}) des Gasgemischs (6) messen (M_{M1}, M_{M2}), und
• die Steuervorrichtung (3) das Gasgemisch (6) auf der Basis der physikalischen Messsignale (C1_{M}, C2_{M}) identifiziert (Ms),
wobei das Verfahren **dadurch gekennzeichnet ist, dass** bei mindestens einem Kalibrierungsschritt (E):
• die Steuervorrichtung (3) die Verlagerung (E_{D}) der Verschlusseinrichtung (42) in die geschlossene Position (P_{F}) derart steuert, dass die Kammer (4) ein geschlossenes Innenvolumen (V_{F}) begrenzt,
• wenn das geschlossene Innenvolumen (V_{F}) frei von Gasgemisch (6) und von Ozon (O) ist, der Photoionisationssensor (2) Ozon (O) in dem geschlossenen Innenvolumen (V_{F}) erzeugt (E_{G}), indem er gemäß einer gegebenen Leistung (Qr) ultraviolette Strahlen (r) sendet, die konfiguriert sind, um den vorhandenen Sauerstoff (D) zu photoionisieren,
• der zu kalibrierende Sensor (1) ein physikalisches Kalibrierungssignal (C1_{E}) des erzeugten Ozons (O) misst (E_{M}),
• die Steuervorrichtung (3) die Abweichung (ε) zwischen dem physikalischen Kalibrierungssignal (C1_{E}) und einem physikalischen Referenzsignal (C1_{I}) als Funktion der gegebenen Leistung (Qr) des Photoionisationssensors (2) berechnet (E_{C}), und
• wenn die Abweichung (ε) größer als eine Referenzabweichung (εref) ist, die Steuervorrichtung (3) eine optimierte Konvertierungsfunktion (f*) für den zu kalibrierenden Sensor (1) auf der Basis der Abweichung (ε) derart bestimmt (Es), dass er geeicht wird.

2. Anwendungsverfahren nach Anspruch 1, wobei bei dem Kalibrierungsschritt (E), wobei das System (S) mindestens eine Vorrichtung zum Entfernen des Gasgemischs (6) in dem Innenvolumen (V) umfasst, die Vorrichtung zum Entfernen das in dem Innenvolumen (V) eventuell verbliebene Gasgemisch (6) vor der Erzeugung (E_{G}) von Ozon (O) entfernt (E_{E}).

3. Anwendungsverfahren nach Anspruch 2, wobei, wobei die Kammer (4) mindestens eine Einlassöffnung (40) und mindestens eine Auslassöffnung (41) umfasst, das Erfassungssystem (S) mindestens eine Lüftungseinrichtung (5) umfasst, die in der Kammer (4) angebracht ist, vorzugsweise im Bereich der Auslassöffnung (41), und mindestens eine Filtereinrichtung (43) des Gasgemischs (6), die im Bereich der Einlassöffnung (40) zwischen einer ausgefahrenen Position und einer eingefahrenen Position beweglich angebracht ist und mit der Lüftungseinrichtung (5) die Vorrichtung zum Entfernen des Gasgemischs (6) bildet, wobei das Entfernen (E_{E}) des Gasgemischs (6) bei dem Kalibrierungsschritt (E_{E}) in dem offenen Innenvolumen (Vo) von der Steuervorrichtung (3) durch gemeinsame Verlagerung der Filtereinrichtung (43) in die ausgefahrene Position (P_{D}) und die Aktivierung der Lüftungseinrichtung (5) derart durchgeführt wird, dass die Aufnahme von Gasgemisch (6) in das offene Innenvolumen (Vo) vermieden und das eventuell verbliebene Gasgemisch (6) aus dem offenen Innenvolumen (Vo) abgeleitet wird.

4. Anwendungsverfahren nach Anspruch 2, wobei, wobei der Photoionisationssensor (2) die Vorrichtung zum Entfernen des Gasgemischs (6) bildet, das Entfernen (E_{E}) des Gasgemischs (6) bei dem Kalibrierungsschritt (E_{E}) in dem geschlossenen Innenvolumen (V_{F}) von dem Photoionisationssensor (2) durch Senden von ultravioletten Strahlen (r) durchgeführt wird, die zum einen konfiguriert sind, um Ozon (O) durch Photoionisation des vorhandenen Sauerstoffs (D) zu erzeugen, um durch Ozonolyse mit dem in dem geschlossenen Innenvolumen (V_{F}) eventuell verbliebenen Gasgemisch (6) zu reagieren und zum anderen, um das Gasgemisch (6) zu photoionisieren.

5. Anwendungsverfahren nach einem der Ansprüche 1 bis 4, wobei bei dem Messschritt (M) das erste physikalische Messsignal (C1_{M}) und das zweite physikalische Messsignal (C2_{M}) gleichzeitig gemessen werden.

6. Anwendungsverfahren nach einem der Ansprüche 1 bis 5, wobei bei mindestens einem Initialisierungsschritt (I):
• die Steuervorrichtung (3) die Verlagerung (I_{D}) der Verschlusseinrichtung (42) in die geschlossene Position (P_{F}) derart steuert, dass die Kammer (4) ein geschlossenes Innenvolumen (V_{F}) begrenzt,
• wenn das geschlossene Innenvolumen (V_{F}) frei von Gasgemisch (6) und von Ozon (O) ist, der Photoionisationssensor (2) Ozon (O) in dem geschlossenen Innenvolumen (V_{F}) erzeugt (E_{G}), indem er gemäß der gegebenen Leistung (Qr) ultraviolette Strahlen (r) sendet, die konfiguriert sind, um den vorhandenen Sauerstoff (D) zu photoionisieren,
• der zu kalibrierende Sensor (1) das physikalische Referenzsignal (C1_{I}) des erzeugten Ozons (O) misst (I_{M}).

7. Chemisches Erfassungssystem mit integrierter Kalibrierung (S) zum Messen eines Gasgemischs (6), wobei das System (S) umfasst:
• eine Kammer (4), die ein Innenvolumen (V) begrenzt und mindestens eine Öffnung (40, 41) zum Versetzen des Gasgemischs (6) und des Innenvolumens (V) in Fluidkommunikation und mindestens eine Verschlusseinrichtung (42) der Öffnung (40, 41) umfasst, die zwischen einer geschlossenen Position (P_{F}), die ein geschlossenes Innenvolumen (V_{F}) begrenzt, und einer geöffneten Position (Po), die ein offenes Innenvolumen (Vo) begrenzt, beweglich angebracht ist,
• mindestens einen chemischen Sensor, bezeichnet als zu kalibrierender Sensor (1), der in dem Innenvolumen (V) der Kammer (4) positioniert ist und ein Messelement (10) umfasst, das konfiguriert ist, um ein elektrisches Signal (U1) zu senden, das von der Menge mindestens einer vorbestimmten chemischen Verbindung (60, 61, O) in dem Innenvolumen (V) abhängt, wobei der zu kalibrierende Sensor (1) ein Berechnungselement (11) eines ersten physikalischen Signals (C1) der chemischen Verbindung (60, 61, O) auf der Basis des elektrischen Signals (U1) und einer eigenen Konvertierungsfunktion (f) des zu kalibrierenden Sensors (1) gemäß der folgenden Gleichung: C1 = f(U1) umfasst, wobei der zu kalibrierende Sensor (1) insbesondere konfiguriert ist, um jeweils bei einem Messschritt (M) und bei einem Kalibrierungsschritt (E) des Anwendungsverfahrens nach einem der Ansprüche 1 bis 6 ein erstes physikalisches Messsignal (C1_{M}) des Gasgemischs (6) in dem offenen Innenvolumen (V_{O}) und ein physikalisches Kalibrierungssignal (C1ε) des Ozons (O) in dem geschlossenen Innenvolumen (V_{F}) zu bestimmen,
• mindestens einen Photoionisationssensor (2), der in dem Innenvolumen (V) positioniert und zum einen konfiguriert ist, um ultraviolette Strahlen (r) derart zu senden, dass mindestens eine vorbestimmte chemische Verbindung (61, D) in dem Innenvolumen (V) photoionisiert wird und zum anderen, um ein zweites physikalisches Signal (C2) zu bestimmen, das von der Menge der photoionisierten chemischen Verbindung (61) abhängt, wobei der Photoionisationssensor (2) insbesondere konfiguriert ist, um bei dem Messschritt (M) ein zweites physikalisches Messsignal (C2_{M}) des Gasgemischs (6) in dem offenen Innenvolumen (Vo) zu bestimmen und um bei dem Kalibrierungsschritt (E) ultraviolette Strahlen (r) gemäß einer gegebenen Leistung (Qᵣ) zu senden, um den Sauerstoff (D) in Ozon (O) in dem geschlossenen Innenvolumen (V_{F}) zu photoionisieren, und
• mindestens eine Steuervorrichtung (3), die konfiguriert ist, um die Verlagerung der Verschlusseinrichtung (42) in die geöffnete Position (Po) beim Messschritt (M) derart zu steuern, dass die Kammer (4) ein offenes Innenvolumen (Vo) begrenzt, und in der geschlossenen Position (P_{F}) bei dem Kalibrierungsschritt (E) derart, dass die Kammer (4) ein geschlossenes Innenvolumen (V_{F}) begrenzt, wobei die Steuervorrichtung (3) konfiguriert ist, um:
• beim Messschritt (M) das Gasgemisch (6) auf der Basis des ersten und des zweiten physikalischen Messsignals (C1_{M}, C2_{M}) zu identifizieren, und
• beim Kalibrierungsschritt (E) die Abweichung (ε) zwischen dem physikalischen Kalibrierungssignal (C1_{E}) und einem physikalischen Referenzsignal (C1_{I}) als Funktion der gegebenen Leistung (Qr) des Photoionisationssensors (2) zu berechnen und, wenn die Abweichung (ε) größer als eine Referenzabweichung (εref) ist, eine optimierte Konvertierungsfunktion (f*) für den zu kalibrierenden Sensor (1) zu bestimmen, dass er geeicht wird.

8. Chemisches Erfassungssystem (S) nach Anspruch 7, wobei die Kammer (4) mindestens eine Einlassöffnung (40), mindestens eine Auslassöffnung (41) und mindestens eine Verschlusseinrichtung (42) der Einlassöffnung (40) und der Auslassöffnung (41) umfasst, um die Zirkulation des Gasgemischs (6) in dem offenen Innenvolumen (V_{O}) zu erleichtern.

9. Chemisches Erfassungssystem (S) nach einem der Ansprüche 7 und 8, umfassend mindestens eine Lüftungseinrichtung (5), die in der Kammer (4), vorzugsweise im Bereich der Auslassöffnung (41), angebracht ist, um die Erneuerung des Gasgemischs (6) in dem offenen Innenvolumen (Vo) zu fördern.

10. Chemisches Erfassungssystem (S) nach einem der Ansprüche 7 bis 9, wobei der zu kalibrierende Sensor (1) in Form eines Halbleitersensors oder eines elektrochemischen Sensors vorliegt.

## Claims

1. A method for operating an integrated calibration chemical sensing system (S) for measuring a gas mixture (6), said system (S) comprising:
- a chamber (4) delimiting an internal volume (V) and comprising at least one opening (40, 41) for fluidly communicating the gas mixture (6) and the internal volume (V) and at least one closure member (42) for said opening (40, 41) movably mounted between a closed position (P_{F}) delimiting a closed internal volume (V_{F}) and an open position (Po) delimiting an open internal volume (Vo),
- at least one chemical sensor, so-called sensor to be calibrated (1), positioned in the internal volume (V) of the chamber (4) and comprising a measurement element (10) configured to emit an electrical signal (U1) that is a function of the amount of at least one predetermined chemical compound (60, 61, O) in the internal volume (V), said sensor to be calibrated (1) comprising a calculation element (11) for a first physical signal (C1) of said chemical compound (60, 61, O) from said electrical signal (U1) and a conversion function (f) specific to said sensor to be calibrated (1) according to the following relationship: C1 = f(U1),
- at least one photoionisation sensor (2) positioned in the internal volume (V), deemed not subject to drift, and configured, on the one hand, to emit ultraviolet rays(r) so as to photoionise at least one predetermined chemical compound (61, D) in the internal volume (V), and on the other hand, to determine a second physical signal (C2) that is a function of the amount of said chemical compound (61) photoionised, and
- at least one drive device (3),
- in which method, during at least one measurement step (M),
- the drive device (3) controls the displacement (MD) of the closure member (42) into the open position (Po) such that the chamber (4) delimits an open internal volume (Vo) into which the gas mixture (6) enters,
- the sensor to be calibrated (1) and the photoionisation sensor (2) measure (M_{M1}, M_{M2}) respectively at least one first physical measurement signal (C1_{M}) and at least one second physical measurement signal (C2_{M}) of the gas mixture (6), and
- the drive device (3) identifies (M_{S}) the gas mixture (6) from the physical measurement signals (C1_{M}, C2_{M}),
- which method is **characterised in that** during at least one calibration step (E):
- the drive device (3) controls the displacement (E_{D}) of the closure member (42) in the closed position (P_{F}) so that the chamber (4) delimits a closed internal volume (V_{F}),
- when the closed internal volume (V_{F}) is free of gas mixture (6) and ozone (O), the photoionisation sensor (2) generates (Ec) ozone (O) in the closed internal volume (V_{F}) by emitting at a given power (Qr) ultraviolet rays (r) configured to photoionise dioxygen (D) present,
- the sensor to be calibrated (1) measures (E_{M}) a physical calibration signal (C1ε) of the ozone (O) generated,
- the drive device (3) calculates (E_{C}) the deviation (ε) between the physical calibration signal (C1ε) and a physical reference signal (C1_{I}), as a function of the given power (Qr) of the photoionisation sensor (2), and
- if the deviation (ε) is greater than a reference deviation (εref), the drive device (3) determines (Es) an optimised conversion function (f*) for the sensor to be calibrated (1) from said deviation (ε), in order to calibrate it.

2. The operating method according to claim 1, wherein during the calibration step (E), the system (S) comprising at least one device for removing the gas mixture (6) in the internal volume (V), the removing device removes (E_{E}) the gas mixture (6) possibly remaining in the internal volume (V) prior to generating (E_{G}) ozone (O).

3. The operating method according to claim 2, wherein the chamber (4) comprises at least one inlet opening (40) and at least one outlet opening (41), the sensing system (S) comprising at least one ventilation member (5) mounted in the chamber (4), preferably at the outlet opening (41), and at least one filtering member (43) for the gas mixture (6) movably mounted at the inlet opening (40) between a deployed position and a retracted position and forming, together with the ventilation member (5), the device for removing the gas mixture (6), removing (Eε) the gas mixture (6) during the calibration step (E_{E}) is implemented in the open internal volume (V_{O}) by the drive device (3) by jointly controlling displacement of the filtering member (43) into the deployed position (P_{D}) and activation of the ventilation member (5), so as to avoid intake of the gas mixture (6) into the open internal volume (Vo) and to discharge any remaining gas mixture (6) from the open internal volume (Vo).

4. The operating method according to claim 2, wherein, the photoionisation sensor (2) forming the device for removing the gas mixture (6), removing (Eε) the gas mixture (6) during the calibration step (E_{E}) is implemented in the closed internal volume (V_{F}) by the photoionisation sensor (2) by emitting ultraviolet rays (r) configured, on the one hand, to generate ozone (O) by photoionising dioxygen (D) present in order to react by ozonolysis with the gas mixture (6) possibly remaining in the closed internal volume (V_{F}), and on the other hand to photoionise said gas mixture (6).

5. The operating method according to any of claims 1 to 4, wherein, during the measurement step (M), the first physical measurement signal (C1_{M}) and the second physical measurement signal (C2_{M}) are simultaneously measured.

6. The operating method according to any of claims 1 to 5, wherein, during at least one initialisation step (I):
- the drive device (3) controls the displacement (I_{D}) of the closure member (42) in the closed position (P_{F}) so that the chamber (4) delimits a closed internal volume (V_{F}),
- when the closed internal volume (V_{F}) is free of gas mixture (6) and ozone (O), the photoionisation sensor (2) generates (I_{G}) ozone (O) in the closed internal volume (V_{F}) by emitting at said given power (Qr) ultraviolet rays (r) configured to photoionise dioxygen (D) present and
- the sensor to be calibrated (1) measures (I_{M}) said physical reference signal (C1_{I}) of ozone (O) generated.

7. An integrated calibration chemical sensing system (S) for measuring a gas mixture (6), said system (S) comprising:
- a chamber (4) delimiting an internal volume (V) and comprising at least one opening (40, 41) for fluidly communicating the gas mixture (6) and the internal volume (V) and at least one closure member (42) for said opening (40, 41) movably mounted between a closed position (P_{F}) delimiting a closed internal volume (V_{F}) and an open position (Po) delimiting an open internal volume (Vo),
- at least one chemical sensor, so-called sensor to be calibrated (1), positioned in the internal volume (V) of the chamber (4) and comprising a measurement element (10) configured to emit an electrical signal (U1) that is a function of the amount of at least one predetermined chemical compound (60, 61, O) in the internal volume (V), said sensor to be calibrated (1) comprising a calculation element (11) for a first physical signal (C1) of said chemical compound (60, 61, O) from said electrical signal (U1) and a conversion function (f) specific to said sensor to be calibrated (1) according to the following relationship: C1 = f(U1), said sensor to be calibrated (1) being in particular configured to determine, during a measurement step (M) and during a calibration step (E) of the operating method according to one of claims 1 to 6 respectively, a first physical measurement signal (C1_{M}) of the gas mixture (6) in the open internal volume (Vo) and a physical calibration signal (C1ε) of ozone (O) in the closed internal volume (V_{F}),
- at least one photoionisation sensor (2) positioned in the internal volume (V) and configured, on the one hand, to emit ultraviolet rays (r) so as to photoionise at least one predetermined chemical compound (61, D) in the internal volume (V), and on the other hand, to determine a second physical signal (C2) which is a function of the amount of said chemical compound (61) photoionised, said photoionisation sensor (2) being in particular configured, during the measurement step (M), to determine a second physical measurement signal (C2_{M}) of the gas mixture (6) in the open internal volume (Vo) and, during the calibration step (E), to emit ultraviolet rays (r) at a given power (Qr) to photoionise dioxygen (D) into ozone (O) in the closed internal volume (V_{F}), and
- at least one drive device (3) configured to control displacement of the closure member (42) in the open position (Po) during the measurement step (M), such that the chamber (4) delimits an open internal volume (Vo), and in the closed position (P_{F}) during the calibration step (E), such that the chamber (4) delimits a closed internal volume (V_{F}), said drive device (3) being configured:
- during the measurement step (M), to identify the gas mixture (6) from the first and second physical measurement signal (C1_{M}, C2_{M}) and,
- in the calibration step (E), to calculate the deviation (ε) between the physical calibration signal (C1ε) and a physical reference signal (C1_{I}), as a function of the given power (Qr) of the photoionisation sensor (2), and, if the deviation (ε) is greater than a reference deviation (εref), determine an optimised conversion function (f*) for the sensor to be calibrated (1), in order to calibrate it.

8. The chemical sensing system (S) according to claim 7, wherein the chamber (4) comprises at least one inlet opening (40), at least one outlet opening (41) and at least one closure member (42) for the inlet opening (40) and the outlet opening (41), in order to facilitate circulation of the gas mixture (6) in the open internal volume (Vo).

9. The chemical sensing system (S) according to any of claims 7 and 8, comprising at least one ventilation member (5) mounted in the chamber (4), preferably at the outlet opening (41), to promote turnover of the gas mixture (6) in the open internal volume (Vo).

10. The chemical sensing system (S) according to any of claims 7 to 9, wherein the sensor to be calibrated (1) is in the form of a semiconductor sensor or an electrochemical sensor.
